# EUROPÄISCHE PATENTANMELDUNG

(11) **EP 4 509 497 A1**
(43) Veröffentlichungstag der Anmeldung: **19.02.2025**
(21) Anmeldenummer: 23191822.8
(22) Anmeldetag: 17.08.2023
(51) Int. Cl.: C07D 213/82, A61K 31/455, A61K 31/616, C07C 65/21

(54) **VERFAHREN ZUR HERSTELLUNG VON ACETYLSALICYLSÄURE-NICOTINSÄUREAMID CO-KRISTALLEN UND ACETYLSALICYLSÄURE- NICOTINSÄUREAMID CO-KRISTALLE MIT VERBESSERTER LÖSLICHKEIT**

(71) Anmelder: Ruhr-Universität Bochum, 44801 Bochum (DE)
(72) Erfinder: Merz, Klaus, 51375 Leverkusen (DE); Schauerte, Carsten, 40723 Hilden (DE); Lamkowski, Laura, 59368 Werne (DE)
(74) Vertreter: Michalski Hüttermann & Partner Patentanwälte mbB

(57) **Zusammenfassung**

Die Erfindung betrifft ein Verfahren zur Herstellung von Acetylsalicylsäure-Nicotinsäureamid-Co-Kristallen, wobei 2-Acetoxybenzoesäure und Pyridin-3-carboxamid in einem Molverhältnis, berechnet als eingesetzte Mol 2-Acetoxybenzoesäure dividiert durch eingesetzte Mol Pyridin-3-carboxamid, von größer oder gleich 0,67 und kleiner oder gleich 1,0 unter Anwendung eines mechanochemischen Verfahrens ohne Ausbildung kovalenter Bindungen zwischen der 2-Acetoxybenzoesäure und dem Pyridin-3-carboxamid miteinander zur Reaktion gebracht werden. Des Weiteren betrifft die vorliegende Erfindung ein Acetylsalicylsäure-Nicotinsäureamid-Addukt.

## Beschreibung

Die Erfindung betrifft ein Verfahren zur Herstellung von Acetylsalicylsäure-Nicotinsäureamid-Co-Kristallen, wobei 2-Acetoxybenzoesäure und Pyridin-3-carboxamid in einem Molverhältnis, berechnet als eingesetzte Mol 2-Acetoxybenzoesäure dividiert durch eingesetzte Mol Pyridin-3-carboxamid, von größer oder gleich 0,67 und kleiner oder gleich 1,0 unter Anwendung eines mechanochemischen Verfahrens ohne Ausbildung kovalenter Bindungen zwischen der 2-Acetoxybenzoesäure und dem Pyridin-3-carboxamid miteinander zur Reaktion gebracht werden. Des Weiteren betrifft die vorliegende Erfindung ein Acetylsalicylsäure-Nicotinsäureamid-Addukt.

2-Acetoxybenzoesäure, auch bekannt als Acetylsalicylsäure kurz ASS, ist seit langem als effektives Arzneimittel in den unterschiedlichsten Indikationen und Darreichungsformen bekannt. ASS wird sowohl als Mono- als auch als Kombipräparat in einer breiten Palette unterschiedlicher Tabletten, beispielsweise in den Ausgestaltungen Brausetabletten, Retardtabletten, Filmtabletten, Sublingualtabletten, Schmelztabletten, Kautabletten angeboten. Daneben findet sich ASS auch in Form von Pulvern, Granulaten, Dragées, Kapseln, Injektionslösungen und Suppositorien. Die Vielfalt an unterschiedlichen Darreichungsformen ist ein Hinweis darauf, dass ASS in vielfältigen chemischen Umgebungen stabil formuliert und mit einer hinreichenden Bioverfügbarkeit eingesetzt werden kann. Formulierungen in wässrigen Umgebungen sind hingegen herausfordernd, da die Löslichkeit von ASS in Wasser sehr begrenzt ist. Aus diesem Grund wird ASS überwiegend in nicht-wässrigen Formulierungen vermarktet.

Auch in nicht-wässrigen Darreichungsformen könnte eine schnellere Löslichkeit vorteilhaft sein, da anzunehmen ist, dass die schnellere Löslichkeit mit einer besseren, d.h. schnelleren Bioverfügbarkeit einhergeht. Zur Steigerung der Effizienz in der medizinischen Anwendung wäre eine solche natürlich äußerst wünschenswert. Weiterhin wünschenswert wäre, wenn diese schnellere Bereitstellung ohne einen zu hohen Anteil an nicht physiologisch wirksamen Substanzen erkauft werden könnte.

Auch in der Patentliteratur finden sich die unterschiedlichsten Ansätze zur Verbesserung der Bioverfügbarkeit und/oder zur Erhöhung der Löslichkeit in wässrigen Medien.

So beschreibt beispielsweise die WO 2022 036 291 A9 ein Verfahren zur Herstellung einer Zusammensetzung, die einen Cokristall umfasst, umfassend:
(A) Erhalten eines pharmazeutischen Wirkstoffs und eines Co-Bildners, um eine physikalische Mischung zu erhalten;
(B) Unterziehen der physikalischen Mischung einem Extrusionsverfahren, um eine Zusammensetzung zu erhalten, die einen Co-Kristall umfasst; wobei der Extrusionsprozess zwei oder mehr Temperaturzonen umfasst.

In der WO2022125869A1 ist ein Verfahren zur Herstellung einer pharmazeutischen Zusammensetzung beschrieben, das Verfahren umfasst:
(A) Erhalten einer Mischung aus einem pharmazeutischen Wirkstoff (API) und einem Coformer;
(B) Unterziehen der Mischung einer dielektrischen Erwärmung, um eine pharmazeutische Zusammensetzung zu erhalten; wobei die pharmazeutische Zusammensetzung mindestens 50 % des API umfasst und der Coformer als Cokristall vorliegt.

Die WO 2008 037 366 A2 betrifft eine neue Form von Acetylsalicylsäure, die als Form AB bezeichnet wird, sowie Verfahren zu ihrer Herstellung und Formulierung, die sie umfasst. Die Erfindung betrifft auch feste Formen von Acetylsalicylsäure Form AB, insbesondere Form AB-A, sowie Verfahren zu ihrer Herstellung und Formulierung, die diese umfassen.

Durch die US4120958A wird ein 2-Acetoxybenzoesäure-Nikotinamid-Komplex bereitgestellt, der verbesserte Wasserlöslichkeits- und Auflösungseigenschaften aufweisen soll und so eine schnelle Absorption der 2-Acetoxybenzoesäure durch die Magen-Darm-Schleimhaut ermöglicht, um außergewöhnliche 2-Acetoxybenzoesäure-Blutspiegel zu erreichen. Der Komplex wird über ein lösemittelbasiertes Verfahren bereitgestellt, wobei das Verfahren sämtlich zu Acetoxybenzoesäure-Nikotinamid-Komplexen mit einer Stöchiometrie von 1:2 führt. Andere als 1:2-Komplexe wurden weder dargestellt noch lassen sie sich nach dem beschriebenen Lösemittelverfahren erhalten.

Derartige aus dem Stand der Technik bekannte Lösungen können noch weiteres Verbesserungspotential bieten. Dies bezieht sich insbesondere auf die Herstellung sehr effizienter Komplexe und insbesondere kristalliner Systeme, welche sich durch eine verbesserte Auflösung und eine optimierte Wirkstoffbeladungen auszeichnen.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren bereitzustellen, welches reproduzierbar zu geordnet aufgebauten ASS-Co-Kristallen mit höheren ASS-Anteilen und verbesserter Auflösung in wässrigen Medien führt. Des Weiteren ist es die Aufgabe der vorliegenden Erfindung verbessert lösliche ASS-Nicotinsäureamid-Co-Kristalle mit verbesserter Stöchiometrie bereitzustellen.

Die Lösung der Aufgabe erfolgt durch die Merkmale der unabhängigen Ansprüche, gerichtet auf das erfindungsgemäße Verfahren sowie die erfindungsgemäßen Co-Kristalle. Bevorzugte Ausgestaltungen der Erfindung sind in den Unteransprüchen, in der Beschreibung oder den Figuren angegeben, wobei weitere in den Unteransprüchen, in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß ist demzufolge ein Verfahren zur Herstellung von Acetylsalicylsäure-Nicotinsäureamid-Co-Kristallen, wobei 2-Acetoxybenzoesäure und Pyridin-3-carboxamid in einem Molverhältnis, berechnet als eingesetzte Mol 2-Acetoxybenzoesäure dividiert durch eingesetzte Mol Pyridin-3-carboxamid, von größer oder gleich 0,67 und kleiner oder gleich 1,0 unter Anwendung eines mechanochemischen Verfahrens ohne Ausbildung kovalenter Bindungen zwischen der 2-Acetoxybenzoesäure und dem Pyridin-3-carboxamid miteinander zur Reaktion gebracht werden. Überraschenderweise wurde festgestellt, dass sich mittels des angegebenen Verfahrens mit hohen Ausbeuten, sehr reproduzierbar in Wasser verbessert lösliche und geordnet aufgebaute Acetylsalicylsäure-Nicotinsäureamid-Co-Kristalle ergeben, welche sich aufgrund der verbesserten Löslichkeit und einem verbesserten Wirkstoff- zu Hilfsstoff-Verhältnis insbesondere für pharmazeutische Anwendungen eignen. Im Bereich der pharmazeutischen Formulierungen kann dieser Komplex insbesondere für orale Applikationen vorteilhaft sein, welche durch die verbesserte Löslichkeit eine schnellere Bioverfügbarkeit zeigen sollten. Das Verfahren ist leicht up-scale fähig und kommt energieschonend ohne einen großen Einsatz von Lösemitteln und ohne große Trockenoperationen aus.

Das erfindungsgemäße Verfahren ist ein Verfahren zur Herstellung von Acetylsalicylsäure-Nicotinsäureamid-Co-Kristallen, wobei die beiden Substanzen: 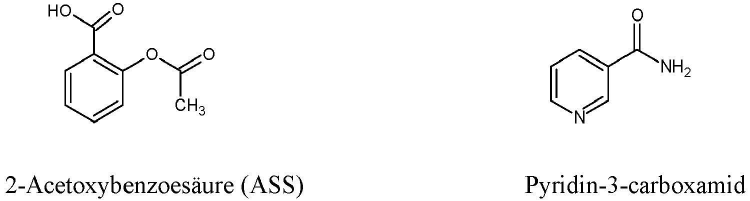 miteinander einen Co-Kristall ausbilden. Das Pyridin-3-carboxamid ist weiterhin unter den Begriffen Nicotinsäureamid, Niacinamid (NA), Pyridin-3-carbonsäureamid bekannt, wobei die Begriffe hier synonym verwendet werden. Die 2-Acetoxybenzoesäure ist weiterhin unter dem Begriff Acetylsalicylsäure bekannt. Auch diese Begriffe werden hier synonym verwendet. Das Verfahren führt dabei nicht nur zu einem Addukt der beiden Substanzen. Es werden geordnet aufgebaute Kristalle mit regelmäßigen Abständen der beiden Substanzen erhalten, welche sich in Pulverdiffraktogrammen durch spezifische Peaks auszeichnen. Dies im Gegensatz zu amorphen Substanzen, welche in Pulverdiffraktogrammen nur breite Halos zeigen.

Das Molverhältnis zwischen 2-Acetoxybenzoesäure und Pyridin-3-carboxamid wird berechnet als eingesetzte Mol 2-Acetoxybenzoesäure dividiert durch eingesetzte Mol Pyridin-3-carboxamid, und ist größer oder gleich 0,67 und kleiner oder gleich 1,0. Über das Verfahren lassen sich Co-Kristalle mit einem hohen Anteil an ASS erhalten. Dieser Anteil ist höher als der Anteil, welcher durch Stand der Technik Verfahren erhältlich ist. Für das Verhältnis wird das zu Beginn der Reaktion vorliegende molare Verhältnis von ASS und NA verwendet. Über dieses Verhältnis können ASS:NA-Co-Kristalle mit einer Stöchiometrie von 1:1 bis zu ca. 1:1, 5 erhalten werden.

Die Co-Kristalle werden unter Anwendung eines mechanochemischen Verfahrens erhalten. In der Mechanochemie können Feststoffe direkt und ohne Lösungsmittel miteinander in Reaktion gebracht werden. Eine mechanochemische Reaktion wird durch die direkte Aufnahme von mechanischer Energie ausgelöst. Die mechanische Kraft, die zur Auslösung einer mechanisch-chemischen Reaktion erforderlich ist, kann das Ergebnis einer Vielzahl von Prozessen sein. Dazu gehören Stöße, Schläge, Druck und Scherkräfte. Im Gegensatz zu global auftretenden Phänomenen wie der Erhitzung sind diese Prozesse lokaler Natur und ermöglichen einzigartige Reaktionswege - einer der Hauptvorteile der Mechanochemie. Die Vielfalt der Phänomene erschwert jedoch eine mechanistische Interpretation der mechanochemischen Reaktionen, da sie in den meisten Fällen gemeinsam auftreten. Mechanochemische Verfahren im Sinne der Erfindung sind insbesondere die Verfahren, welche sich durch eine intensive mechanische Bearbeitung der Probe auszeichnen. Übliche Reaktionszeiten liegen dabei in einem Bereich von größer oder gleich 5 Minuten, weiter bevorzugt größer oder gleich 10 Minuten, des Weiteren bevorzugt größer oder gleich 20 Minuten, in denen beide Reaktionspartner unter mechanischer Belastung miteinander reagieren können. Bevorzugt kann die Reaktion bei Rumtemperatur oder darüber durchgeführt werden. Der mechanische Energieeintrag während der mechanochemischen Reaktion kann bevorzugt größer oder gleich 40 Watt, weiter bevorzugt größer oder gleich 60 Watt und weiterhin bevorzugt größer oder gleich 80 Watt bezogen auf 0,5 mmol Edukte betragen.

Die Co-Kristalle werden ohne Ausbildung kovalenter Bindungen zwischen der 2-Acetoxybenzoesäure und dem Pyridin-3-carboxamid miteinander zur Reaktion gebracht. Das zur Reaktion bringen der beiden Edukte erfolgt also ohne Ausbildung einer direkten kovalenten Bindung zwischen den Reaktionspartnern. Es werden assoziative, im Sinne elektrostatischer, Wechselwirkungen ausgebildet, welche zu der Bildung des ASS-NA-Adduktes beitragen. Diese Art der Wechselwirkung lässt sich durch eine Kombination von ¹H- und ¹³C-NMR Untersuchungen nachweisen. ¹H-NMR Untersuchungen in Lösung zeigen das Vorliegen der Edukte und ¹³C solid state NMR-Untersuchungen zeigen eine Verschiebung der Signale.

In einer bevorzugten Ausführungsform des Verfahrens kann das eingesetzte Molverhältnis von 2-Acetoxybenzoesäure zu Pyridin-3-carboxamid größer oder gleich 0,83 und kleiner oder gleich 1,0 betragen. Zum Erhalt eines möglichst ASS-reichen Co-Kristalls hat sich die Verwendung möglichst kleiner Mengen an NA als besonders geeignet herausgestellt. Es werden lagerstabile ASS-NA-Co-Kristalle erhalten, welche sich durch eine deutlich verbesserte Auflösung in wässriger Umgebung auszeichnen. Die Stöchiometrie des Co-Kristalls kann im Rahmen üblicher Messungenauigkeit für jedes der beiden Edukte um +/- 2,5 mol % abweichen. Die quantitative Konzentrationsbestimmung der beiden Edukte erfolgt dabei nach dem Fachmann bekannten Verfahren, beispielsweise über HPLC.

Innerhalb einer weiter bevorzugten Ausgestaltung des Verfahrens kann das eingesetzte Molverhältnis von 2-Acetoxybenzoesäure zu Pyridin-3-carboxamid 1,0 betragen. Zum Erhalt eines möglichst ASS-reichen Co-Kristalls hat sich die Verwendung möglichst kleiner Mengen an NA als besonders geeignet herausgestellt. Es werden lagerstabile ASS-NA-Co-Kristalle erhalten, welche sich durch eine deutlich verbesserte Auflösung in wässriger Umgebung auszeichnen. Die Stöchiometrie des Co-Kristalls kann im Rahmen üblicher Messungenauigkeit für jedes der beiden Edukte um +/- 2,5 mol % abweichen. Die quantitative Konzentrationsbestimmung der beiden Edukte erfolgt dabei nach dem Fachmann bekannten Verfahren, beispielsweise über HPLC.

Innerhalb eines weiter bevorzugten Aspektes des Verfahrens kann, das zur Reaktion bringen in Gegenwart eines Lösungsmittels erfolgen, wobei das Gewichtsverhältnis zwischen Lösungsmittel und der Summe aus 2-Acetoxybenzoesäure und Pyridin-3-carboxamid, berechnet nach Gewicht Lösungsmittel dividiert durch das Gewicht (2-Acetoxybenzoesäure + Pyridin-3-carboxamid), größer oder gleich 0 % und kleiner oder gleich 50 % beträgt. Das zur Reaktionbringen mittels eines mechanochemischen Verfahrens kann unter Zugabe sehr geringer Mengen Lösemittel erfolgen. Die Lösemittelmengen sind dabei so gering, dass sich keine "Auflösung" der Edukte im Lösemittel ergibt. Dieses Verfahren wird als "solvent-drop assistet" bezeichnet und führt in den beanspruchten Mengenbereichen dazu, dass die Substanzen nicht als "nass" bezeichnet werden können. Bevorzugt kann das Gewichtsverhältnis größer oder gleich 5 % und kleiner oder gleich 40% und weiter bevorzugt größer oder gleich 15 % und kleiner oder gleich 30 % betragen. Diese geringen Mengen ermöglichen die Herstellung homogener Co-Kristalle. Größeren Mengen können zu rein physikalischen Mischungen oder zu Mischkristallen führen.

Nach einer bevorzugten Charakteristik des Verfahrens kann das Lösemittel ein nicht-protisches Lösemittel sein. Insbesondere die Verwendung geringer Mengen nicht protischer Lösemittel in "solvent-drop assistet"-Verfahren kann dazu beitragen, dass Acetylsalicylsäure-Nicotinsäureamid-Co-Kristalle erhalten werden können, welche sich durch ein besonders günstiges ASS-Verhältnis auszeichnen. Es lassen sich Co-Kristalle mit einem Edukt-Verhältnis nahe um 1:1 erhalten, welche eine besonders verbesserte Löslichkeit zeigen. Bevorzugt kann das Gewichtsverhältnis der protischen Lösemittel zum Gewicht der Edukte größer oder gleich 5 % und kleiner oder gleich 40% und weiter bevorzugt größer oder gleich 15 % und kleiner oder gleich 30 % betragen.

In einer weiter bevorzugten Ausführungsform des Verfahrens kann das Lösemittel Ethylacetat sein. Ethylacetat hat sich als ein besonders geeignetes Lösemittel zur Herstellung möglichst ASS-reicher und homogener Acetylsalicylsäure-Nicotinsäureamid-Co-Kristalle herausgestellt. Es werden sehr lagerstabile Co-Kristalle erhalten, welche sich durch eine besonders verbesserte Löslichkeit in wässrigen Medien auszeichnen. Bevorzugt kann das Gewichtsverhältnis der protischen Lösemittel zum Gewicht der Edukte größer oder gleich 5 % und kleiner oder gleich 40 % und weiter bevorzugt größer oder gleich 20 % und kleiner oder gleich 30 % betragen.

Innerhalb eines bevorzugten Aspektes des Verfahrens kann das zur Reaktion bringen in Abwesenheit eines Lösungsmittels erfolgen. Überraschend wurde gefunden, dass homogene Co-Kristalle mit einem besonders hohen Anteil an ASS ohne Verwendung von Lösemitteln in mechanochemischen Verfahren erhalten lassen. Neben den Vereinfachungen in der Herstellung entfällt an dieser Stelle auch das Abtrocknen von Lösemitteln und eine Gefahr von Produktverunreinigungen durch Restlösemittel wird gänzlich vermieden.

In einer weiter bevorzugten Ausführungsform des Verfahrens kann das zur Reaktion bringen mittels einer Kugelmühle erfolgen, wobei das Kugel-zu-Pulver-Volumen-Verhältnis, berechnet aus dem Volumen der Kugeln dividiert durch das Volumen der 2-Acetoxybenzoesäure und des Pyridin-3-carboxamids, größer oder gleich 0,9 und kleiner oder gleich 1,1 beträgt.

Die Herstellung der Acetylsalicylsäure-Nicotinsäureamid-Co-Kristalle mittels einer Kugelmühle kann zu Co-Kristallen mit einem besonders hohem ASS-Anteil führen. Die Herstellung ist sehr reproduzierbar und im oben angegebenen Kugel-zu-Pulver-Volumen-Verhältnis lassen sich die Umsetzungen sehr genau steuern. Bevorzugt kann die Umsetzung ohne Zugabe eines Lösemittels erfolgen. Bevorzugt kann durch dieses Verfahren ein 1:1 Acetylsalicylsäure-Nicotinsäureamid-Co-Kristall erhalten werden. Bevorzugt kann das ASS zu NA-Verhältnis im Kristall 1:1 betragen.

Des Weiteren erfindungsgemäß ist ein Acetylsalicylsäure-Nicotinsäureamid-Addukt, wobei das Molverhältnis von 2-Acetoxybenzoesäure zu Pyridin-3-carboxamid, berechnet als Mol 2-Acetoxybenzoesäure dividiert durch Mol Pyridin-3-carboxamid, größer oder gleich 0,67 und kleiner oder gleich 1,0 beträgt. Die erfindungsgemäßen Acetylsalicylsäure-Nicotinsäureamid-Co-Kristalle zeichnen sich durch einen hohen Anteil an ASS aus und zeigen insbesondere in wässrigen Medien eine verbesserte Löslichkeit. Die Co-Kristalle sind lagerstabil und können mit einem sehr geringen oder aber auch ohne Lösemittelanteil erhalten werden.

Des Weiteren erfindungsgemäß ist ein Acetylsalicylsäure-Nicotinsäureamid-Addukt erhalten über das erfindungsgemäße Verfahren, wobei das Molverhältnis von 2-Acetoxybenzoesäure zu Pyridin-3-carboxamid, berechnet als Mol 2-Acetoxybenzoesäure dividiert durch Mol Pyridin-3-carboxamid, größer oder gleich 0,67 und kleiner oder gleich 1,0 beträgt. Die erfindungsgemäßen Acetylsalicylsäure-Nicotinsäureamid-Co-Kristalle zeichnen sich durch einen hohen Anteil an ASS aus und zeigen insbesondere in wässrigen Medien eine verbesserte Löslichkeit. Die Co-Kristalle sind lagerstabil, sehr homogen aufgebaut und können mit einem sehr geringen oder aber auch ohne Lösemittelanteil erhalten werden. Bevorzugt kann der Restlösemittelanteil, ermittelt über HPLC, kleiner oder gleich 0,5 Gew-%, weiter bevorzugt kleiner oder gleich 0,25 Gew-% und kleiner oder gleich 0,1 Gew-% betragen.

Im Rahmen einer weiterhin bevorzugten Ausgestaltung des Acetylsalicylsäure-Nicotinsäureamid-Addukts kann das Molverhältnis von 2-Acetoxybenzoesäure zu Pyridin-3-carboxamid 1,0 betragen. Die Co-Kristalle können sich durch einen besonders hohen Anteil an der pharmazeutisch wirksamen Substanz ASS auszeichnen. Insbesondere kann das Verhältnis zwischen dem ASS und dem NA 1:1 betragen, wobei Abweichungen in diesem Verhältnis im Messbereich von +/- 2 Gew.-% pro Edukt zulässig sein können. Die Mengen und dadurch auch das Mengenverhältnis können über HPLC-Methoden bestimmt werden.

Innerhalb einer weiter bevorzugten Ausgestaltung des Acetylsalicylsäure-Nicotinsäureamid-Addukts kann das Acetylsalicylsäure-Nicotinsäureamid-Addukt in einem DSC-Thermogramm mit einer Aufheizrate von 5 K/min in einem Temperaturbereich von größer oder gleich 0°C und kleiner oder gleich 120 °C nur einen Schmelzpeak aufweisen, wobei der Schmelzpeak im Bereich von größer oder gleich 80 °C und kleiner oder gleich 10 0°C liegt. Die erfindungsgemäßen Acetylsalicylsäure-Nicotinsäureamid-Co-Kristalle können sich durch eine sehr gleichmäßige Beschaffenheit und eine homogene Verteilung der Co-Kristalle auszeichnen. Als Folge dessen wird auch nur ein Schmelzpeak im oben angegebenen Temperaturintervall detektiert. Dies ist ein Hinweis, dass das Herstellverfahren sehr kontrolliert und geeignet ist nur eine definierte Produkspezies zu erhalten. Dies im Gegensatz zu DSC-Messungen an anderen Co-Kristallen, welche unter nicht kontrollierten Herstellungsbedingungen mehrere unterschiedliche DSC-Peaks aufweisen können. Bevorzugt kann der DSC-Peak eine Halbwertsbreite von kleiner oder gleich 15 °C, weiter bevorzugt von kleiner oder gleich 10 °C und kleiner oder gleich 8 °C aufweisen. Diese Halbwertsbreiten der DSC-Peaks deuten auf ein möglichst homogenen Acetylsalicylsäure-Nicotinsäureamid-Co-Kristall hin.

Innerhalb eines weiter bevorzugten Aspektes des Acetylsalicylsäure-Nicotinsäureamid-Addukts kann das Acetylsalicylsäure-Nicotinsäureamid-Addukt kristallin sein und in Form eines Acetylsalicylsäure-Nicotinsäureamid-Co-Kristalls vorliegen, wobei der Co-Kristall in einem Pulver-Diffraktogramm (Cu-Kα1 = 1,54059 Ä) einen Peak bei 10,5 (+/- 0,2) 2Theta (°) aufweist. Neben dem hohen Anteil an ASS können sich die vorliegenden Co-Kristalle durch ein spezifisches Kristallgitter auszeichnen, welches auf eine spezielle und geordnete Ausrichtung des ASS und des NA schließen lässt.

Nach einer bevorzugten Charakteristik des Acetylsalicylsäure-Nicotinsäureamid-Addukts kann das Acetylsalicylsäure-Nicotinsäureamid-Addukt kristallin sein und in Form eines Acetylsalicylsäure-Nicotinsäureamid-Co-Kristalls vorliegen, wobei der Co-Kristall in einem Pulver-Diffraktogramm (Cu-Kα1 = 1,54059 Ä) Peaks bei 25,8 (+/- 0,2), 10,5 (+/- 0,2) und 25,1 (+/- 0,2) 2Theta (°) aufweist. Neben dem hohen Anteil an ASS können sich die vorliegenden Co-Kristalle durch ein spezifisches Kristallgitter auszeichnen, welches auf eine spezielle und geordnete Ausrichtung des ASS und des NA schließen lässt.

In einer weiter bevorzugten Ausführungsform des Acetylsalicylsäure-Nicotinsäureamid-Addukts kann das Addukt in einem ¹³C-Festkörper-NMR-Spektrum mindestens Signale bei 174,8 (+/- 0,3), 172,8 (+/- 0,3) und 124,0 (+/- 0,3) ppm aufweisen. Die erfindungsgemäßen Acetylsalicylsäure-Nicotinsäureamid-Co-Kristalle können sich durch ein spezifisches ¹³C-Festkörper-NMR-Spektrum auszeichnen. Ohne durch die Theorie gebunden zu sein, scheinen die Signale bei 175 ppm und 172,8 ppm dem ASS im Acetylsalicylsäure-Nicotinsäureamid-Co-Kristall zuzuordnen zu sein. Weiterhin kann sich das ¹³C-Festkörper-NMR-Spektrum durch Signale bei 174.8, 172.8, 151.5, 130.1 ppm auszeichnen. Weiterhin kann sich das ¹³C-Festkörper-NMR-Spektrum durch Signale bei 174.8, 172.8, 151.5, 130.1, 129.4, 126.8 ppm auszeichnen. Weiterhin kann sich das ¹³C-Festkörper-NMR-Spektrum durch Signale bei 174.8, 172.8, 169.7, 169.0, 167.2, 153.0, 151.5, 130.1, 129.4, 126.8, 124.0 ppm auszeichnen.

Innerhalb eines bevorzugten Aspektes des Acetylsalicylsäure-Nicotinsäureamid-Addukts kann das Acetylsalicylsäure-Nicotinsäureamid-Addukt in einem FTIR-Spektrum eine N-H-Bande bei 3425,1 (+/- 1,0) cm⁻¹ und eine OH-Bande bei 3189,9 cm⁻¹ aufweisen. Die erfindungsgemäßen Acetylsalicylsäure-Nicotinsäureamid-Co-Kristalle können sich insbesondere durch spezifische FTIR-Banden auszeichnen. Ohne durch die Theorie gebunden zu sein, lassen sich die Absorptionen bei ca. 3425 cm⁻¹ der NH-Bande des NA zuordnen. Gleiches gilt für die Zuordnung der Absorption bei 3189 cm⁻¹ zur OH-Bande des ASS. Weiter bevorzugt kann das FTIR-Banden bei 3425,1, 3187,9, 1775,23; weiter bevorzugt bei 3425,1, 3187,9, 1775,23, 1750,2; weiter bevorzugt bei 3425,1, 3187,9, 1775,23, 1750,2, 1690,4 und weiter bevorzugt bei 3425,1, 3187,9, 1775,23, 1750,2, 1690,4 und 1625,8 cm⁻¹ zeigen.

Innerhalb einer weiter bevorzugten Ausgestaltung des Acetylsalicylsäure-Nicotinsäureamid-Addukts kann das Acetylsalicylsäure-Nicotinsäureamid-Addukt kristallin sein und in Form eines Acetylsalicylsäure-Nicotinsäureamid-Co-Kristalls vorliegen, wobei der Co-Kristall in einem Pulver-Diffraktogramm (Cu-Kα1 = 1,54059 Ä) Peaks bei 25,8 (+/- 0,2) und bei 10,6 (+/- 0,2) 2Theta (°) aufweist.

Innerhalb einer weiter bevorzugten Ausgestaltung des Acetylsalicylsäure-Nicotinsäureamid-Addukts kann das Acetylsalicylsäure-Nicotinsäureamid-Addukt kristallin sein und in Form eines Acetylsalicylsäure-Nicotinsäureamid-Co-Kristalls vorliegen, wobei der Co-Kristall in einem Pulver-Diffraktogramm (Cu-Kα1 = 1,54059 Ä) Peaks bei 25,8 (+/- 0,2), 10,6 (+/- 0,2) und bei 27,3 (+/- 0,2) 2Theta (°) aufweist.

Weitere Vorteile und vorteilhafte Ausgestaltungen der erfindungsgemäßen Gegenstände werden durch die Figuren veranschaulicht und in den nachfolgenden Beispielen erläutert. Dabei ist zu beachten, dass die Figuren nur beschreibenden Charakter haben und nicht dazu gedacht sind, die Erfindung in irgendeiner Form einzuschränken.

Es zeigen die
- Fig. 1: eine ¹H-NMR-spektroskopische Aufnahme einer Lösung eines erfindungsgemäßen Acetylsalicylsäure-Nicotinsäureamid-Co-Kristalls;
- Fig. 2: eine ¹³C-Festkörper-NMR-spektroskopische Aufnahme einer Lösung eines erfindungsgemäßen Acetylsalicylsäure-Nicotinsäureamid-Co-Kristalls;
- Fig. 3: ein DSC-Thermogramm eines erfindungsgemäßen Acetylsalicylsäure-Nicotinsäureamid-Co-Kristalls;
- Fig.4: ein FTIR-Spektrogramm eines erfindungsgemäßen Acetylsalicylsäure-Nicotinsäureamid-Co-Kristalls;
- Fig.5: ein PXRD-Diffraktogramm eines erfindungsgemäßen Acetylsalicylsäure-Nicotinsäureamid-Co-Kristalls;
- Fig.6: eine TGA-Aufnahme eines erfindungsgemäßen Acetylsalicylsäure-Nicotinsäureamid-Co-Kristalls;
- Fig.7: einen Vergleich der Löslichkeiten von ASS und ASS in einem erfindungsgemäßen Acetylsalicylsäure-Nicotinsäureamid-Co-Kristall nach 1 h.

### Beispiele

### I. Analytik

### La DSC

### DSC der Ausgangsstoffe

Die DSC-Daten wurden mit der Netzsch Phoenix DSC 204 F1 ermittelt. Ungefähr 1-2 mg des Ausgangsmaterials wurden in eine DSC-Schale gegeben und das Gewicht aufgezeichnet. Für die Analyse wurden gewellte Schalen mit einer Lochblende verwendet, und die Proben wurden unter Stickstoffatmosphäre mit einer Geschwindigkeit von 5 K/min bis zu einer Endtemperatur von 150 °C erhitzt. Acetylsalicylsäure zeigt einen ersten Schmelzpeak bei 139,5 °C und Nicotinamid einen ersten Schmelzpeak bei 130,1 °C.

### Nachweis des Acetylsalicylsäure-Nicotinamid-Co-Kristalls

Die DSC-Daten des Co-Kristalls werden 5 Tage nach der Synthese durchgeführt. Zur Messung wird der Co-Kristall gemahlen. Die DSC-Daten wurden mit einem Netzsch Phoenix DSC 204 F1 ermittelt. Ungefähr 1-2 mg der reinen gemahlenen Probe wurden in eine DSC-Schale gegeben und das Gewicht aufgezeichnet. Die Proben wurden unter Stickstoffatmosphäre mit einer Geschwindigkeit von 5 K/min bis zu einer Endtemperatur von 200 °C erhitzt. Der Acetylsalicylsäure-Nicotinamid-Cokristall zeigt einen Schmelzpeak bei 91 °C.

### I.b Pulver-Röntgendiffraktometrie

### D2

Für die Pulver-Röntgendiffraktometrie (PXRD) wurden die Proben in Standard-Glaskapillaren (Ø = 0,7 mm) eingefüllt. Die Messungen wurden bei Raumtemperatur mit einem D8 Bruker Advance Diffraktometer (Cu-Kα1 = 1,54059 Ä, Johansson Primärstrahlmonochromator, positionsempfindlicher Detektor) im Transmissionsmodus mit Rotation der Probe durchgeführt. Die Daten wurden in einem 2Theta-Bereich von 1,5-50° erfasst. Die Röhrenspannung und der Strom betrugen 40 kV bzw. 40 mA.

### I.c TGA

Die TG-Analysen wurden mit einem Perkin Elmer Thermogravimetric Analyzer Pyris 6 TGA durchgeführt. Ungefähr 10-15 mg der Probe wurden in eine tarierte Schale gegeben und in den TG-Ofen eingewogen. Die Proben wurden in Stickstoff mit einer Geschwindigkeit von 10 °C/min bis zu einer Endtemperatur von 350 °C erhitzt.

### I.d FTIR

FT-IR-Spektren wurden für die Ausgangsmaterialien und den Acetylsalicylsäure-Nicotinamid-Co-Kristall mit einem Thermo Scientific Nicolet iS10 in ATR-Geometrie aufgenommen. Jedes Spektrum repräsentiert 32 co-addierte Scans mit einer Auflösung von 4 cm⁻¹. Der Messbereich beträgt 600 - 4000 cm⁻¹.

### I.e NMR

¹H-NMR-Spektren wurden mit einem Bruker Advance DRX 400-Spektrometer (bei 400 MHz) bei Raumtemperatur in deuteriertem Lösungsmittel (d6-DMSO) aufgenommen. Die Informationen über die chemische Verschiebung δ sind in ppm angegeben, bezogen auf die Bestrahlungsfrequenz. Das Signal des deuterierten Lösungsmittels wird als interner Standard verwendet.

### I.f ¹³C-Festkörper-NMR

¹³C-Festkörper-Spektrum wurde mit einem Bruker DSX400MAS (bei 100 MHz) bei Raumtemperatur aufgenommen.

### I.g Löslichkeitsbestimmungen

Es wurden 15 mL gesättigter wässriger Proben mit Niederschlag in 20 mL-Fläschchen mit dem Acetylsalicylsäure-Nicotinsäureamid-Co-Kristall (1:1), ASS und NIC angesetzt. Die Proben wurden bei Raumtemperatur mit einem Magnetrührer homogenisiert. Nach 1 h und 2 h werden von jeder Probe 4-mal ca. 1 mL Lösung entnommen (4-fach Bestimmung) und durch ein Vorspritzfilter in ein leeres 4 mL Fläschchen überführt. Die Fläschchen werden dann bis zur Verdampfung des Lösemittels offen stehengelassen. Die Fläschchen wurden leer, mit der gesättigten Lösung und nach dem Verdampfen gewogen. Die Löslichkeit in Masseprozent ergibt sich über die Differenz aus Gewicht nach dem Verdampfen und dem Leegewicht.

### II. Herstellung der Co-Kristalle

### II.1 Kugelmühle

Acetylsalicylsäure und Nicotinamid werden im Verhältnis 1:1 verwendet (90 mg Acetylsalicylsäure: 61 mg Nicotinamid). Die gewogenen Verbindungen werden in ein 4-mL-Fläschchen gegeben und das gleiche Volumen an Mahlkugeln wird hinzugefügt. Diese Kugeln sind aus Al₂O₃ (Durchmesser ø 1-1,5 mm). Das Mahlen erfolgt 30 Minuten lang bei höchster Frequenz im Bereich von 3000 bis 3600 Umdrehungen pro Minute. Der Energieeintrag liegt bei ca. 90 W.

### II.2 Kugelmühle (lösemittelunterstützt)

Acetylsalicylsäure und Nicotinamid werden im Verhältnis 1:1 verwendet (90 mg Acetylsalicylsäure: 61 mg Nicotinamid). Mit 40 µL Ethylacetat werden die gewogenen Verbindungen in einem 4-mL-Fläschchen gegeben und das gleiche Volumen an Mahlkugeln zugegeben. Diese Kugeln sind aus Al₂O₃ (Durchmesser ø 1-1,5 mm). Das flüssigkeitsunterstützte Mahlen wird 30 Minuten lang bei höchster Frequenz im Bereich von 3000 bis 3600 Umdrehungen pro Minute durchgeführt. Der Energieeintrag liegt bei ca. 90 W.

### III. Ergebnisse

Die Ergebnisse der Acetylsalicylsäure-Nicotinsäureamid-Co-Kristalle wurden an Acetylsalicylsäure-Nicotinsäureamid-Co-Kristallen erhalten, welche über einen trockenen Kugelmühlenprozess erhalten wurden.

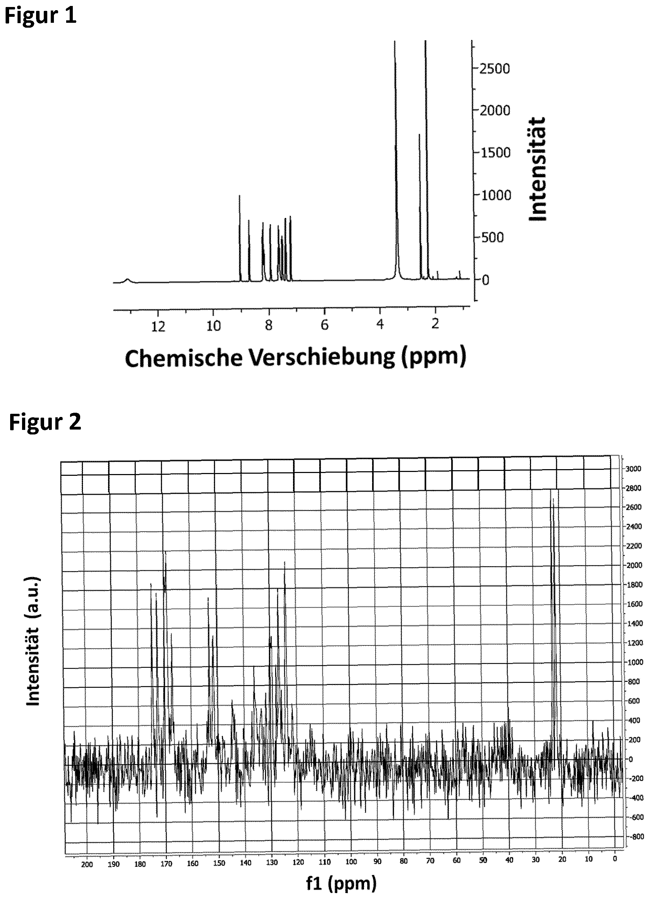

Die Figur 1 zeigt eine ¹H-NMR-spektroskopische Aufnahme einer Lösung eines erfindungsgemäßen Acetylsalicylsäure-Nicotinsäureamid-Co-Kristalls ASS:NA-Verhältnis von 1:1. Die Zuordnung der einzelnen ¹H-NMR-Signale ergeben sich aus der Figur 1 von links nach rechts zu mit nach folgender Zuordnung: 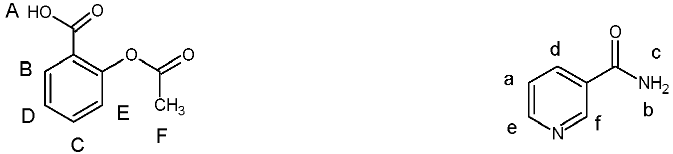 zu A (>12 ppm), f (9 ppm), e, (c, d), B, (C, b), a, D, E und F bei 2,25 ppm. Der DSMO-Peak liegt bei 2,5 ppm. Der Peak bei ca. 3,3 ppm ist nicht zugeordnet.

Die Figur 2 zeigt eine ¹³C-Festkörper-NMR-spektroskopische Aufnahme einer Lösung eines erfindungsgemäßen Acetylsalicylsäure-Nicotinsäureamid-Co-Kristalls mit einem ASS:NA-Verhältnis von 1:1. Die wichtigste Zuordnung eines Signales ergibt sich zu 174,8 ppm für die -COOH-Gruppe. Aus dem ¹³C-Festkörper-NMR lässt sich erkennen, dass es im Festkörper nicht um eine physikalische Mischung aus Acetylsalicylsäure und Nicotinsäureamid handelt.

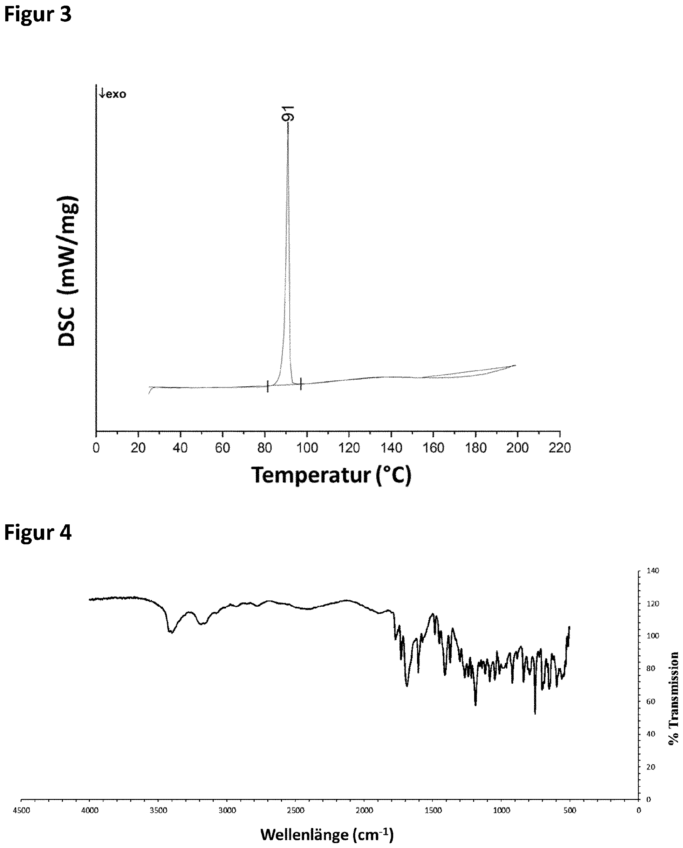

Die Figur 3 zeigt ein DSC-Thermogramm eines erfindungsgemäßen Acetylsalicylsäure-Nicotinsäureamid-Co-Kristalls mit einem ASS:NA-Verhältnis von 1:1. Es ist nur ein gut definierter Phasenübergang im Messbereich detektierbar. Dies deutet darauf hin, dass die hergestellten Acetylsalicylsäure-Nicotinsäureamid-Co-Kristalle homogene Kristalle ohne große Anteile weiterer Phasen darstellen.

Die Figur 4 zeigt ein FTIR-Spektrogramm eines erfindungsgemäßen Acetylsalicylsäure-Nicotinsäureamid-Co-Kristalls mit einem ASS:NA-Verhältnis von 1:1. Das FTIR-Spektrogramm zeigt insbesondere eine Verschiebung der O-H, und C=O Banden der Acetylsalicylsäure von 1418 nach 1405 cm⁻¹ und 1757 nach 1790 cm⁻¹ und 1690 nach 1700 sowie der N-H Bande des Nicotinsäureamids von 3380 nach 3450, 3500 cm⁻¹ und 3280 nach 3250 cm⁻¹.

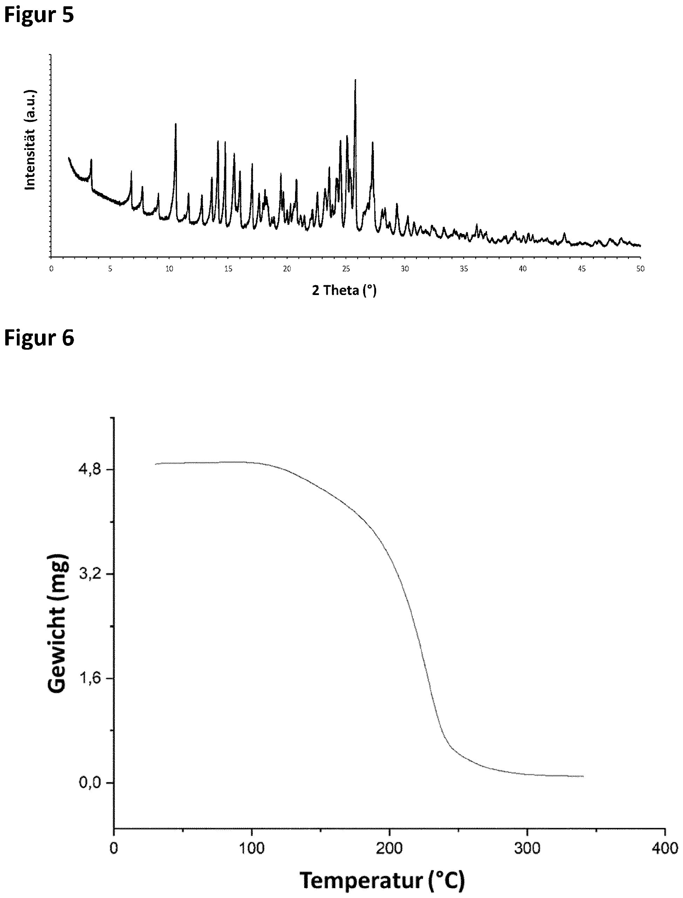

Die Figur 5 zeigt ein PXRD-Diffraktogramm eines erfindungsgemäßen Acetylsalicylsäure-Nicotinsäureamid-Co-Kristalls mit einem ASS:NA-Verhältnis von 1:1. Das PXRD-Diffraktogramm ist ein Beleg dafür, dass es sich um eine echte kristalline Verbindung, einen Co-Kristall, von ASS und NA handelt.

Die Figur 6 zeigt eine TGA-Aufnahme eines erfindungsgemäßen Acetylsalicylsäure-Nicotinsäureamid-Co-Kristalls mit einem ASS:NA-Verhältnis von 1:1. Ebenso wie im DSC ist in der TGA nur ein definierter Übergang zu erkennen. Der Übergang kann dem Schmelzen des Acetylsalicylsäure-Nicotinsäureamid-Co-Kristalls zugeordnet werden.

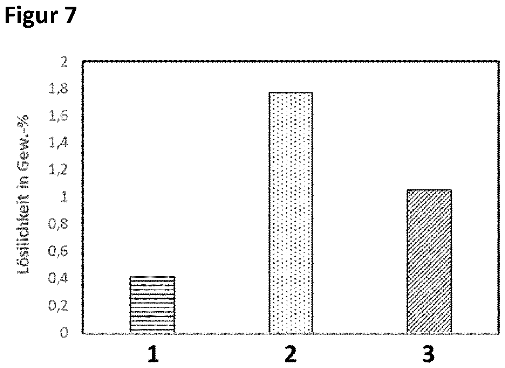

Die Figur 7 zeigt einen Vergleich der Löslichkeiten von ASS (1) und ASS (3) in einem erfindungsgemäßen Acetylsalicylsäure-Nicotinsäureamid-Co-Kristall (2) mit einem ASS:NA-Verhältnis von 1:1. Es lässt sich deutlich erkennen, dass über die Einbindung in den Co-Kristall die ASS-Löslichkeit um einen Faktor von ca. 2,5 gesteigert werden kann. Dies erkennt man an der aufgelösten Gewichtsmenge (1) im Vergleich zu (3). Der Balken (2) entspricht der aufgelösten Menge des Co-Kristalls.

Die einzelnen Daten aus den Löslichkeitsversuchen ergeben sich zu:

| Löslichkeit | Nach 1 h [ω %] | Nach 2 h [ω %] |
|---|---|---|
| Acetylsalicylsäure | 0,414 | 0,426 |
| Co-Kristall | 1,768 | 1,777 |
| ASS im Co-Kristall | 1,054 | 1,059 |

Es lässt sich erkennen, dass sich zwischen den 1 h und 2 h Daten keine großartigen Änderungen in Bezug auf die Löslichkeit ergeben. Insofern scheinen auf dieser Zeitskala keine kinetischen Effekte eine Rolle zu spielen.

Die Versuche zur Herstellung eines Acetylsalicylsäure-Nicotinsäureamid-Co-Kristalls mit höheren NA-Verhältnissen wurden wiederholt. Ab einem Verhältnis von ASS zu NA von über 1:1,5 sind keine reinen Acetylsalicylsäure-Nicotinsäureamid-Co-Kristalle mehr erhältlich. Insbesondere bei hohen Verhältnissen, beispielsweise von 1:2 ergeben sich größtenteils undefinierte physikalische Mischungen der beiden Edukte und keine definierten Co-Kristalle. Das hier erfindungsgemäß vorgestellte Verfahren ist also insbesondere dazu geeignet, sehr definierte und sehr homogene Co-Kristalle mit definierter Struktur und Stöchiometrie zu erzeugen. Dies im Gegensatz zu anderen Verfahren oder Verfahrensdurchführungen, welche nur sehr inhomogene Verbindungen erhalten. Diese inhomogenen Verbindungen zeigen zudem eine ungünstige Stöchiometrie. Ohne durch die Theorie gebunden zu sein, scheint die Inhomogenität der Kristalle insbesondere eine Folge der erhaltenen Stöchiometrie zu sein.

## Patentansprüche

1. Verfahren zur Herstellung von Acetylsalicylsäure-Nicotinsäureamid-Co-Kristallen, **dadurch gekennzeichnet, dass** 2-Acetoxybenzoesäure und Pyridin-3-carboxamid in einem Molverhältnis, berechnet als eingesetzte Mol 2-Acetoxybenzoesäure dividiert durch eingesetzte Mol Pyridin-3-carboxamid, von größer oder gleich 0,67 und kleiner oder gleich 1,0 unter Anwendung eines mechanochemischen Verfahrens ohne Ausbildung kovalenter Bindungen zwischen der 2-Acetoxybenzoesäure und dem Pyridin-3-carboxamid miteinander zur Reaktion gebracht werden.

2. Verfahren nach Anspruch 1, wobei das eingesetzte Molverhältnis von 2-Acetoxybenzoesäure zu Pyridin-3-carboxamid größer oder gleich 0,83 und kleiner oder gleich 1,0 beträgt.

3. Verfahren nach Anspruch 1, wobei das eingesetzte Molverhältnis von 2-Acetoxybenzoesäure zu Pyridin-3-carboxamid 1,0 beträgt.

4. Verfahren nach einem der vorhergehenden Ansprüche, wobei das zur Reaktion bringen in Gegenwart eines Lösungsmittels erfolgt, wobei das Gewichtsverhältnis zwischen Lösungsmittel und der Summe aus 2-Acetoxybenzoesäure und Pyridin-3-carboxamid, berechnet nach Gewicht Lösungsmittel dividiert durch das Gewicht (2-Acetoxybenzoesäure + Pyridin-3-carboxamid), größer oder gleich 0 % und kleiner oder gleich 50 % beträgt.

5. Verfahren nach Anspruch 4, wobei das Lösemittel ein nicht-protisches Lösemittel ist.

6. Verfahren nach Anspruch 4 oder 5, wobei das Lösemittel Ethylacetat ist.

7. Verfahren nach einem der Ansprüche 1 bis 3, wobei das zur Reaktion bringen in Abwesenheit eines Lösungsmittels erfolgt.

8. Verfahren nach einem der vorhergehenden Ansprüche, wobei das zur Reaktion bringen mittels einer Kugelmühle erfolgt, wobei das Kugel-zu-Pulver-Volumen-Verhältnis, berechnet aus dem Volumen der Kugeln dividiert durch das Volumen der 2-Acetoxybenzoesäure und des Pyridin-3-carboxamids, größer oder gleich 0,9 und kleiner oder gleich 1,1 beträgt.

9. Acetylsalicylsäure-Nicotinsäureamid-Addukt, **dadurch gekennzeichnet, dass** das Molverhältnis von 2-Acetoxybenzoesäure zu Pyridin-3-carboxamid, berechnet als Mol 2-Acetoxybenzoesäure dividiert durch Mol Pyridin-3-carboxamid, größer oder gleich 0,67 und kleiner oder gleich 1,0 beträgt.

10. Acetylsalicylsäure-Nicotinsäureamid-Addukt nach Anspruch 9, wobei das Molverhältnis von 2-Acetoxybenzoesäure zu Pyridin-3-carboxamid 1,0 beträgt.

11. Acetylsalicylsäure-Nicotinsäureamid-Addukt nach einem der Ansprüche 9 oder 10, wobei das Acetylsalicylsäure-Nicotinsäureamid-Addukt in einem DSC-Thermogramm mit einer Aufheizrate von 5 K/min in einem Temperaturbereich von größer oder gleich 0 °C und kleiner oder gleich 120 °C nur einen Schmelzpeak aufweist, wobei der Schmelzpeak im Bereich von größer oder gleich 80 °C und kleiner oder gleich 100 °C liegt.

12. Acetylsalicylsäure-Nicotinsäureamid-Addukt nach einem der Ansprüche 9 bis 11, wobei das Acetylsalicylsäure-Nicotinsäureamid-Addukt kristallin ist und in Form eines Acetylsalicylsäure-Nicotinsäureamid-Co-Kristalls vorliegt, wobei der Co-Kristall in einem Pulver-Diffraktogramm (Cu-Kα1 = 1,54059 Ä) einen Peak bei 10,5 (+/- 0,2) 2Theta (°) aufweist.

13. Acetylsalicylsäure-Nicotinsäureamid-Addukt nach einem der Ansprüche 9 bis 11, wobei das Acetylsalicylsäure-Nicotinsäureamid-Addukt kristallin ist und in Form eines Acetylsalicylsäure-Nicotinsäureamid-Co-Kristalls vorliegt, wobei der Co-Kristall in einem Pulver-Diffraktogramm (Cu-Kα1 = 1,54059 Ä) Peaks bei 25,8 (+/- 0,2), 10,5 (+/- 0,2) und 25,1 (+/-0,2) 2Theta (°) aufweist.

14. Acetylsalicylsäure-Nicotinsäureamid-Addukt nach einem der Ansprüche 9 bis 13, wobei das Addukt in einem ¹³C-Festkörper-NMR-Spektrum mindestens Signale bei 174,8 (+/-0,3), 172,8 (+/- 0,3) und 124,0 (+/- 0,3) ppm aufweist.

15. Acetylsalicylsäure-Nicotinsäureamid-Addukt nach einem der Ansprüche 9 bis 14, wobei das Acetylsalicylsäure-Nicotinsäureamid-Addukt in einem FTIR-Spektrum eine N-H-Bande bei 3425,1 (+/- 1,0) cm⁻¹ und eine OH-Bande bei 3189,9 cm⁻¹ aufweist.
